# NOUVEAU FASCICULE DE BREVET EUROPEEN

(11) **EP 3 512 606 B2**
(45) Date de publication et mention de la décision concernant l'opposition: **02.12.2026**
(45) Mention de la délivrance du brevet: 19.07.2023
(21) Numéro de dépôt: 17777616.8
(22) Date de dépôt: 14.09.2017
(51) Int. Cl.: A61Q 17/00, A61Q 19/00, A61K 8/99, A61K 36/064

(54) **NOUVELLE UTILISATION COSMÉTIQUE ET/OU NUTRACEUTIQUE OU DERMATOLOGIQUE D'UN EXTRAIT DE LEVURE**
KOSMETISCHE UND/ODER NUTRAZEUTISCHE ODER DERMATOLOGISCHE VERWENDUNG EINES HEFEEXTRAKTS
COSMETIC AND/OR NUTRACEUTIC OR DERMATOLOGICAL USE OF A YEAST EXTRACT

(30) Priorité: 15.09.2016 FR 1658653
(43) Date de publication de la demande: 24.07.2019
(73) Titulaire: BASF Beauty Care Solutions France SAS, 69007 Lyon (FR)
(72) Inventeur: ANDRE-FREI, Valérie, 69420 Ampuis (FR); LEOTY-OKOMBI, Sabrina, Cedex 816, 38090 Vaulx Milieu (FR); MOUSSOU, Philippe, 54510 Tomblaine (FR)
(74) Mandataire: Cabinet Becker et Associés
(86) Numéro de dépôt international: PCT/FR2017/052447
(87) Numéro de publication internationale: WO 2018/051021

(56) Documents cités:
- WO-A1-2008/064893
- WO-A1-2012/107244
- WO-A2-2006/136420
- CA-A1- 2 354 656
- JP-B2- 3 869 880
- US-A1- 2016 008 269
- DATABASE GNPD [online] MINTEL; August 2016 (2016-08-01), PROBIOKASHI, SOUTH AFRICA: "Probiotic Pet feed Additive", XP002767460, Database accession no. 4200237
- JULIEN SENESCHAL ET AL: "Human Epidermal Langerhans Cells Maintain Immune Homeostasis in Skin by Activating Skin Resident Regulatory T Cells", IMMUNITY., vol. 36, no. 5, 1 May 2012 (2012-05-01), US, pages 873 - 884, XP055233359, ISSN: 1074-7613, DOI: 10.1016/j.immuni.2012.03.018

## Description

L'invention a pour objet l'utilisation non-thérapeutique cosmétique et/ou nutraceutique ou dermatologique, par voie topique et/ou orale, d'un extrait de levure de *Saccharomyces cerevisiae* qui est un hydrolysat fermenté par Lactobacillus plantarum pour augmenter la flore microbienne commensale cutanée et/ou mucosale et notamment la teneur en *Staphylococcus epidermidis* au niveau de la peau et/ou des muqueuses. L'invention a aussi pour objet l'extrait qui est un hydrolysat de *S. cerevisae* fermenté par Lactobacillus plantarum pour son utilisation pharmacéutique.

La peau est le plus grand organe de l'organisme humain. Elle possède un rôle majeur de protection vis à vis des agressions extérieures telle que les agressions de l'environnement, les agressions climatiques, la pollution, les allergènes, les germes pathogènes. La peau représente en outre un écosystème complexe sur lequel prolifèrent plusieurs types de microorganismes tels que bactéries et champignons. Ces microorganismes constituent la flore cutanée, aussi nommée flore microbienne cutanée. On distingue la flore dite commensale constituée des microorganismes proliférant classiquement sur une peau saine, de façon permanente en puisant leurs nutriments sur la peau, de la flore transitoire, présente sur la peau dans des conditions anormales, par exemple par contact avec des éléments souillés, et qui peut devenir pathogène en cas de prolifération. Parmi les microorganismes de la flore commensale cutanée, on citera en particulier la souche *Staphylococcus epidermidis.* On trouve cette souche notamment au niveau du visage, chez des peaux saines, laquelle participe au maintien de l'équilibre de la flore commensale cutanée. En revanche, la souche *Staphylococcus aureus* peut être considérée comme faisant partie de la flore transitoire cutanée potentiellement néfaste pour la peau humaine. *Staphylococcus aureus* peut en effet être à l'origine d'un déséquilibre microbien au niveau de la flore cutanée et induit une inflammation qui s'accompagne notamment de symptômes d'inconfort, de démangeaisons et de sécheresse, et peut être impliqué dans des pathologies cutanées telle que la dermatite atopique.

Des solutions cosmétiques ou dermatologiques sont déjà connues pour agir sur la flore microbienne cutanée. En revanche, leurs actions sont souvent antiseptiques. Ces modes d'action ne sont ainsi pas ciblés car ils ne sont pas dirigés contre une souche de microorganisme spécifique. Il existe des solutions permettant d'agir sur la flore microbienne cutanée en ciblant un groupe comme les bactéries dans le domaine des ingrédients pharmaceutiques. On citera à titre d'exemple les antibiotiques. Toutefois, les antibiotiques présentent l'inconvénient de ne pas toujours être tolérés après administration orale. Ils induisent en outre le développement de résistances. Leur coût de fabrication peut s'avérer élevé et la mise au point de leur développement est souvent longue. Par conséquent, il existe un besoin important dans le domaine de la cosmétique et de la dermatologie de fournir des ingrédients actifs sur la flore microbienne cutanée, notamment par action ciblée sur une souche microbienne particulière, qui soient facilement disponibles et ne possèdent pas les inconvénients ou effets secondaires précédemment décrits.

De manière inattendue, les inventeurs ont découvert qu'un extrait de levure de *S. cerevisiae* avait la capacité d'augmenter la flore microbienne commensale cutanée et/ou mucosale, en particulier en augmentant la teneur en *Staphylococcus epidermidis* et préférentiellement en augmentant le ratio de la teneur de la souche *Staphylococcus epidermidis* par rapport à la souche *Staphylococcus aureus* au niveau de la peau et/ou des muqueuses. Les inventeurs ont ainsi découvert que l'utilisation d'un extrait de levure de *S*. *cerevisiae* répondait au problème technique précédemment énoncé sans les inconvénients de l'art antérieur.

La souche *Saccharomyces cerevisiae* est largement connue et utilisée dans le domaine alimentaire depuis l'Antiquité. C'est une souche employée en oenologie pour la fermentation alcoolique dans la fabrication du vin et de la bière, mais surtout pour la préparation du pain, ce qui explique qu'elle est souvent qualifiée de « levure de boulanger ».

Cette souche a déjà été utilisée dans le domaine cosmétique. Ainsi, l'utilisation cosmétique d'une combinaison d'hespéridine et d'un microorganisme probiotique pouvant être la souche *S. cerevisiae* pour prévenir et/ou renforcer la fonction barrière de la peau est connue dans la demande WO2009031106.

D'autre part, la demande WO2013122932 divulgue une méthode pour augmenter le nombre de microorganismes anaérobiques et/ou aérobiques faisant partie de la flore commensale cutanée, par l'intermédiaire de substances parmi lesquelles les galactooligosaccharides et/ou microorganismes prébiotiques. La souche *Saccharomyces cerevisiae* n'est pas divulguée dans cette demande comme souche prébiotique et l'objet de la présente invention n'est pas l'utilisation de *S*. *cerevisiae* comme prébiotique. Le terme de « prébiotique » désigne dans cette demande un microorganisme administré par voie orale et qui stimule la croissance ou permet le maintien de la présence de souches bactériennes commensales présentes dans le tractus digestif. De manière générale, les prébiotiques sont des oligosaccharides ou polysaccharides, substrats pour les microorganismes.

En outre, l'utilisation de microorganismes probiotiques c'est-à-dire vivants, parmi laquelle la souche *S*. *cerevisiae,* est divulguée dans cette demande WO2013122932 pour l'amélioration de l'apparence de la peau, l'augmentation de l'épaisseur d'une ou plusieurs de ses couches, son élasticité, sa fermeté, sa souplesse. Ces caractéristiques ne sont pas liées à la flore commensale cutanée ni mucosale. Cette demande ne divulgue pas non plus un extrait de *S*. *cerevisiae* ayant la capacité d'augmenter la teneur de la souche *S*. *epidermidis* au niveau de la peau.

La demande FR2872047A1 divulgue l'utilisation de microorganisme probiotique et/ou l'une de ses fractions et/ou l'un de ses métabolites, ledit microorganisme pouvant être *S. cerevisiae,* pour prévenir et/ ou traiter les peaux sensibles, associées ou non aux peaux sèches, en tout cas réactives. La demande CA 2 354 656 A1 décrit un immunostimulant, en particulier un stimulant du système immunitaire de la peau qui peut être un extrait de Saccharomyces cerevisiae. Toutefois, aucune de ces demandes ne divulguent l'utilisation d'un extrait qui est un hydrolysat fermenté de la souche *S. cerevisiae,* encore moins par *Lactobacillus plantarum,* ou son utilisation pour diminuer les manifestations inesthétiques et/ou inconfortables des peaux et/ou muqueuses sensibles, ou pour le traitement des peaux irritées. L'extrait spécifique selon l'invention a en outre été décrit par la Demanderesse et commercialisé sous le nom Relipidium^{™} pour son effet sur l'hydratation de la peau et sa capacité à augmenter la synthèse des lipides au niveau de la peau.

Ainsi à la connaissance de la demanderesse, aucun document de l'art antérieur ne décrit l'utilisation d'un extrait qui est un hydrolysat fermenté de *S. cerevisae* par Lactobacillus plantarum pour augmenter la flore microbienne commensale cutanée et/ou mucosale, en particulier la teneur en *Staphylococcus epidermidis* au niveau de la peau et/ou des muqueuses, notamment la muqueuse labiale, ni pour augmenter le ratio de la teneur de *Staphylococcus epidermidis* par rapport à la teneur de *Staphylococcus aureus* au niveau de la peau et/ou des muqueuses.

L'avantage de l'extrait selon la présente invention est qu'il est très facile d'en obtenir à échelle industrielle. En outre, il est déjà utilisé dans les domaines cosmétiques et dermatologiques et a montré son innocuité sur la peau humaine. L'extrait est toléré par tout type de peaux, ne provoque pas d'allergies et n'est pas toxique.

Un premier objet de la présente invention concerne donc l'utilisation non-thérapeutique cosmétique et/ou nutraceutique ou dermatologique d'un extrait de *S. cerevisae* qui est un hydrolysat fermenté par Lactobacillus plantarum pour augmenter la flore microbienne commensale cutanée et/ou mucosale et/ou pour augmenter le ratio de la teneur d'une ou plusieurs souches microbiennes commensales par rapport à la teneur d'une ou plusieurs souches microbiennes pathogènes au niveau de la peau et/ou des muqueuses.

On entend au sens de la présente invention par « utilisation cosmétique et/ou nutraceutique et/ou composition cosmétique et/ou nutraceutique » une utilisation et/ou une composition non pharmaceutique, c'est-à-dire qui ne nécessite pas de traitement thérapeutique, c'est-à-dire destinée à toute zone de peau, incluant le cuir chevelu, et/ou muqueuses, dite saines. On entend par « peau et/ou muqueuse saine » tout ou partie d'une zone de peau incluant le cuir chevelu et/ou muqueuse saine, notamment humaine, sur laquelle est appliquée l'extrait selon l'invention et dite « non pathologique » par un dermatologue, c'est à dire ne présentant pas d'infection, de cicatrice, de maladie ou d'affection cutanée telle que candidose, impétigo, psoriasis, eczéma, acné ou dermatite, ou de plaies ou de blessures et/ou autres dermatoses. Il s'agit donc d'une utilisation de l'extrait sur zone de peau et/ou de muqueuse de sujets notamment humains qualifiés de « normaux » par un dermatologue, de façon particulière de sujets non immunodéficients. On entend au sens de la présente invention par « peau » la peau de tout ou partie du corps humain choisi parmi les jambes, les pieds, les aisselles, les mains, les cuisses, le ventre, le décolleté, le cou, les bras, le torse, le dos, la muqueuse labiale, le visage et/ou le cuir chevelu, avantageusement le décolleté et/ou le visage, encore avantageusement le visage.

On entend d'autre part par « muqueuse », la muqueuse oculaire, la muqueuse vaginale, la muqueuse uro-génitale, la muqueuse anale, la muqueuse nasale et/ou la muqueuse labiale, et préférentiellement la muqueuse labiale. Au sens de la présente invention, le terme « muqueuse » n'inclut pas la muqueuse buccale ni la muqueuse gingivale, qui présentent une flore microbienne différente de la flore microbienne cutanée.

L'utilisation selon l'invention peut être par voie topique et/ou voie orale, préférentiellement topique. Au sens de la présente invention, on entend par « voie topique », l'application locale directe et/ou la vaporisation de l'ingrédient sur la surface de la peau incluant le cuir chevelu et/ou les muqueuses. On entend par « voie orale » l'administration orale de l'ingrédient, notamment à titre de complément alimentaire dans le cadre d'une utilisation nutraceutique de l'extrait selon l'invention.

L'extrait de *S*. *cerevisae* selon l'invention est topiquement et/ou oralement acceptable. Au sens de la présente invention, on entend par « topiquement et/ou oralement acceptable », un ingrédient adapté à une application respectivement par voie topique et/ou orale, non toxique, non irritant pour la peau et/ou les muqueuses et/ou le cuir chevelu, n'induisant pas de réponse allergique, qui n'est pas instable sur le plan chimique.

On entend au sens de la présente invention par « augmenter la flore microbienne commensale cutanée et/ou mucosale » augmenter la teneur au niveau de la peau et/ou des muqueuses d'une ou plusieurs souches de microorganismes commensaux choisi parmi les champignons, les levures, les bactéries, préférentiellement les bactéries, présent ou apporté sur la peau ou les muqueuses notamment humaines et dont l'action est bénéfique pour la peau et/ou les muqueuses tels que *Staphylococcus hominis, S. warneri, S. capitis, S. epidermidis,* préférentiellement *Staphylococcus epidermidis.* Ainsi au sens de la présente invention, l'utilisation de la souche *S. epidermidis* n'est pas pour lutter contre des infections, notamment celles pouvant être induites chez des humains immunodéficients.

On entend donc au sens de la présente invention par « souche ou flore commensale » une souche ou flore bénéfique pour la peau et/ou les muqueuses, qui n'est pas ou ne devient pas pathogène pour la peau et/ou les muqueuses.

On entend par ailleurs par « microorganisme pathogène » un microorganisme présent sur la peau de façon non permanente et induisant ou pouvant induire des altérations non pathologiques de la peau telles que sécheresse cutanée non pathologique, peau squameuse et craquelée, démangeaisons, rougeurs, sensibilité, peau à tendance atopique, sans processus inflammatoire, voir des affections pathologiques, tels que *Candida albicans,* les malassezia, les Streptocoques, les Staphylocoques et de façon particulière *Staphylococcus aureus.*

Dans un mode de réalisation avantageux de l'invention, on entend par « augmenter le ratio de la teneur d'une ou plusieurs souches microbiennes commensales par rapport à la teneur d'une ou plusieurs souches microbiennes pathogènes au niveau de la peau et/ou des muqueuses » augmenter le ratio de la teneur de la souche *Staphylococcus epidermidis* par rapport à la teneur de la souche *Staphylococcus aureus* détectées au niveau de peaux traitées en présence de l'extrait selon l'invention d'un ratio d'au moins 1,2, avantageusement d'au moins 1,5, encore avantageusement d'au moins 2. Avantageusement, la mesure du ratio est effectuée au bout de 14 jours de traitement dans les conditions décrites dans l'exemple 3a), en présence de l'ingrédient cosmétique tel que décrit dans l'exemple 2.

Plusieurs méthodes peuvent être utilisées pour mesurer la teneur des souches de microorganismes au niveau de la peau et/ou des muqueuses, parmi lesquelles un comptage des colonies présentes sur la peau ou les muqueuses, une mesure *in vitro* par densité optique après récupération d'échantillons contenant les souches ou une mesure par PCR. Avantageusement, la teneur des microorganismes est évaluée en mesurant la concentration en ADN de chacune des 2 souches *S*. *epidermidis* et *S*. *aureus* présentes sur la peau du visage de femmes. Encore avantageusement, l'ADN est mesuré par PCR quantitative.

Au sens de la présente invention, l'utilisation de l'extrait de *S. cerevisiae* selon l'invention pour augmenter la flore microbienne commensale cutanée et/ou mucosale est pour prévenir l'apparition de souches de microorganismes pathogènes au niveau de la peau et/ou des muqueuses. On entend ici par « prévenir » empêcher l'apparition de souches pathogènes au niveau de la peau et/ou des muqueuses, et non pas traiter. Il s'agit donc bien d'une utilisation cosmétique et pas d'un traitement thérapeutique. Ladite utilisation est aussi pour améliorer l'homéostasie cutanée, avantageusement épidermique, encore avantageusement au niveau de la peau et/ou des muqueuses. On entend au sens de la présente invention par « homéostasie cutanée » le maintien de l'équilibre entre les fonctions d'échange, de prolifération et différenciation cellulaire.

Préférentiellement, l'utilisation de l'extrait de *S. cerevisiae* pour augmenter la flore microbienne commensale cutanée et/ou mucosale n'est pas pour améliorer la fonction barrière de la peau ni pour augmenter l'hydratation de la peau et/ou des muqueuses ou pour le traitement des peaux sèches. L'extrait selon l'invention, est un hydrolysat fermenté de S. *cerevisae* par *L. plantarum,* qui diminue les manifestations inesthétiques et/ou inconfortables des peaux et/ou muqueuses sensibles. On entend par « peaux et/ou muqueuses sensibles » des peaux et/ou muqueuses de nature sensible et/ou sensibilisées par des agents agressants tels que la pollution, des conditions climatiques défavorables, le tabac, le stress, tel que le stress hormonal, sans impliquer de réaction inflammatoire ou allergique, et donc ne nécessitant pas un traitement thérapeutique.

On entend au sens de la présente invention par « diminuer les manifestations inesthétiques », diminuer les manifestations visibles et superficielles des peaux sensibles, c'est-à-dire notamment des peaux rendues non homogènes, c'est-à-dire présentant un teint inhomogène. On entend par « diminuer les manifestations inconfortables » des peaux et/ou muqueuses sensibles, diminuer les sensations de démangeaisons, tiraillements, tension, rugosité, échauffement de la peau et/ou des muqueuses.

Ainsi, l'utilisation de l'extrait de *S. cerevisiae* n'est pas une utilisation en tant qu'ingrédient anti-microbien, le qualificatif d'« ingrédient antimicrobien » s'entendant ici d'un ingrédient qui induit la mort du/des microbe(s) concerné(s) ou une inhibition directe de leur croissance, le terme « microbe » englobant à la fois les bactéries, les champignons, en particulier pathogènes. L'extrait selon l'invention a donc un rôle de régulation de la flore microbienne cutanée, en favorisant l'apparition d'une souche commensale plutôt que d'une souche pathogène, avantageusement de la souche commensale *S. epidermidis* plutôt que la souche *S. aureus.* Ainsi selon l'invention, l'extrait de *S. cerevisiae* n'induit pas la mort de *S. aureus,* tel que démontré dans l'exemple 3b) de la présente description. L'utilisation de l'extrait selon l'invention est donc pour augmenter la teneur en *S. epidermidis* au niveau de la peau et/ou des muqueuses.

L'extrait selon la présente invention est obtenu à partir de la levure *Saccharomyces cerevisiae.* L'extrait peut être tout ou partie de la levure inactivée ou partie de la levure vivante, il peut comprendre la paroi, la membrane, le contenu cellulaire uniquement et/ou tout mélange de chacune de ces parties. Selon l'invention, l'extrait n'est pas la forme vivante de *S. cerevisiae.*

De manière préférentielle, l'extrait peut être obtenu par une première étape d'autolyse, de plasmolyse et/ou par une ou plusieurs hydrolyses enzymatiques et/ou chimiques, préférentiellement enzymatiques. L'hydrolyse enzymatique est préférentiellement effectuée avec des protéases choisies parmi les protéases à cystéine telles que la papaine, les caspases, les protéases à sérine telle que la trypsine, les protéases acides telle la pepsine. Avantageusement, l'hydrolyse est effectuée en présence d'une protéase à cystéine telle que la papaine (Vukasinovic Milic *et al.,* 2007 ; Dolinska *et al.,* 2012). L'hydrolyse pourra être effectuée dans l'eau ou dans un milieu aqueux tamponné, préférentiellement dans l'eau uniquement. L'hydrolyse pourra être effectuée à pH contrôlé.

Préférentiellement selon la présente invention, l'extrait de *S. cerevisiae* ne contient pas de polysaccharide du type mannane, glucomannane, arabinogalactan, galactomannane, ou encore des oligosaccharides, en particulier pas de galactooligosaccharides, ou l'un quelconque de leur(s) hydrolyzat(s) sous la forme de disaccharide, trisaccharide, trétrasaccharide, pentasaccharide, hexasaccharide et/ou l'un quelconque de leur(s) mélange(s).

Dans un mode de réalisation avantageux de la présente invention, le procédé d'obtention de l'extrait de *S*. *cerevisiae* comprend une étape de séparation des fractions solubles et insolubles, et élimination de la fraction insoluble. La fraction soluble est récupérée notamment par centrifugation. Préférentiellement, l'extrait de *S. cerevisiae* correspond à la fraction soluble de la levure obtenu après hydrolyse enzymatique.

Selon un mode de réalisation avantageux de l'invention, l'extrait selon l'invention ne contient pas d'hydroxyisoleucine. L'extrait selon l'invention pourra contenir des sucres solubles résiduels, en particulier de type monosaccharide, ou non.

L'extrait de *S. cerevisiae* obtenu correspond préférentiellement à un hydrolysat, avantageusement soluble.

L'extrait est ensuite fermenté par une souche de bactérie lactique, ladite souche de bactérie étant choisie parmi une souche de la famille des Lactobacillaceae et du genre *Lactobacillus* telle que *L. plantarum*. Selon l'invention, la souche utilisée est *Lactobacillus plantarum.* La fermentation pourra être conduite durant une période de 2 à 24 heures, préférentiellement 12 heures, sous agitation ou non, préférentiellement sous agitation. Dans un mode de réalisation particulièrement avantageux, la fermentation est conduite jusqu'à épuisement du milieu nutritif contenant du glucose.

Une ou plusieurs filtrations peuvent ensuite être mises en oeuvre, avantageusement, au moins une filtration stérile (0,22µm) est incluse dans le procédé d'obtention de l'extrait. Avantageusement ainsi, la souche *L. plantarum,* n'est plus présente dans l'extrait de *S. cerevisiae* après filtration. Selon l'invention, l'extrait de *S. cerevisiae* est donc un hydrolysat fermenté par *L. plantarum.* Préférentiellement, l'hydrolysat de *S. cerevisiae* ne contient plus de fragment, de lysat, d'autolysat ou de composés, notamment de peptides, provenant de *L. plantarum* après filtration.

Ainsi, dans un mode de réalisation préférentiel de l'invention, l'extrait de *S. cerevisiae* est obtenu comme suit : une quantité de 3,5 % en poids de poudre de la levure *S. cerevisiae* inactivée, c'est-à-dire non vivante, par rapport au poids total de poudre et de solvant, est solubilisée dans l'eau comme solvant puis hydrolysée en présence de papaine à une température de 60°C durant une période de 72 heures sous agitation puis le milieu est centrifugé (Vukasinovic Milic *et al.,* 2007). Le surnageant obtenu est ensuite incubé en présence de la souche *L. plantarum* jusqu'à épuisement du milieu nutritif contenant du glucose. L'extrait brut ainsi fermenté obtenu est filtré (0,22µm), tel que décrit dans l'exemple 1a).

Dans un second mode avantageux de réalisation de l'invention, l'extrait de *S. cerevisiae* est obtenu à partir d'une quantité de 5 % en poids de poudre de la levure *S. cerevisiae* inactivée, c'est-à-dire non vivante, par rapport au poids total de poudre et de solvant, solubilisée dans l'eau comme solvant, puis hydrolysé en présence de lyticase à une température de 55°C durant une période de 72 heures sous agitation puis le milieu est centrifugé (Vukasinovic Milic *et al*., 2007). Le surnageant obtenu est ensuite incubé en présence de la souche *L. plantarum* jusqu'à épuisement du milieu nutritif contenant du glucose. L'extrait brut fermenté est filtré (0,22µm) dans les conditions décrites dans l'exemple 1b).

Dans un troisième mode de réalisation avantageux de l'invention, l'extrait de *S. cerevisiae* est obtenu à partir d'une quantité de 3,5% en poids de poudre de la levure *S. cerevisiae* inactivée, c'est-à-dire non vivante, par rapport au poids total de poudre et de solvant, solubilisée dans l'eau comme solvant, et soumise à autolyse, sous agitation et à une température de 52°C durant une période de 72 heures (Vukasinovic Milic *et al.,* 2007). Après centrifugation, le surnageant obtenu est incubé en présence de la souche *L. plantarum* jusqu'à épuisement du milieu nutritif contenant du glucose. L'extrait brut ainsi fermenté est filtré (0,22µm) dans les conditions décrites dans l'exemple 1c).

Préférentiellement selon l'invention, l'hydrolysat fermenté de *S. cerevisiae* obtenu ne contient pas de polysaccharide de type mannane, glucomannane, arabinogalactan, galactomannane, ou encore d'oligosaccharides, en particulier pas de galactooligosaccharides, ou seulement sous forme de traces.

L'extrait de *S. cerevisiae* ainsi obtenu selon l'invention pourra être dissous dans un solvant notamment polaire, en particulier choisi parmi l'eau, un alcool, un polyol, un glycol, tel que le pentylène glycol, le caprylyl glycol, le butylène glycol, l'hexylène glycol ou un de leurs mélanges. Avantageusement, l'extrait est dissous dans un mélange hydroglycolique, préférentiellement un mélange d'eau et de glycol choisi parmi le butylène glycol, le pentylène glycol et leurs mélanges.

De façon avantageuse, la solution aqueuse dans laquelle est solubilité l'extrait de *S. cerevisiae* selon l'invention contient entre 0,1 et 30% de butylène glycol, avantageusement entre 5% et 25%, encore avantageusement entre 1 0% et 25% en poids par rapport au poids total de la solution aqueuse, tel que décrit dans l'exemple 2.

De façon particulière, la solution aqueuse dans laquelle est solubilité l'extrait de *S. cerevisiae* selon l'invention contient en outre entre 0,01 et 40% de pentylène glycol, avantageusement entre 1% et 25% en poids par rapport au poids total de la solution aqueuse, encore avantageusement l'extrait selon l'invention contient entre 5 % et 10 % de pentylène glycol.

L'objet de l'invention concerne donc l'utilisation d'un extrait de *S. cerevisiae,* qui est un hydrolysat fermenté de *S. cerevisiae* par *L. plantarum,* pour augmenter la flore microbienne commensale cutanée et/ou mucosale et/ou pour augmenter le ratio de la teneur de *S. epidermidis* par rapport à la teneur de *S. aureus* au niveau de la peau et/ou des muqueuses.

L'extrait de *S. cerevisae* selon l'invention peut être utilisé seul sous forme d'ingrédient actif cosmétique et/ou nutraceutique ou contenu dans une composition cosmétique et/ou nutraceutique.

Un autre objet de l'invention concerne donc l'utilisation de l'extrait de *S. cerevisae* contenu dans une composition cosmétique et/ou nutraceutique, laquelle comprend par ailleurs au moins un excipient cosmétiquement et/ou nutraceutiquement acceptable. On entend par « excipient acceptable » tout véhicule ou solvant cosmétiquement ou nutraceutiquement adapté à l'utilisation en contact avec la peau, inclus le cuir chevelu, et/ou les muqueuses humaines ou par voie orale sans toxicité, incompatibilité, instabilité, réponse allergique, ou leurs équivalents, indue.

Dans un mode de réalisation de l'invention, l'extrait est contenu dans la composition cosmétique à une concentration en poids de 1×10⁻⁴ % à 10% en poids par rapport au poids total de la composition, avantageusement de 1×10⁻³ % à 3%, encore avantageusement de 0,01% à 3% en poids par rapport au poids total de la composition. La composition peut être formulée sous forme de solution, aqueuse ou huileuse, de crème, sérum, de gel aqueux ou de gel huileux, notamment en pot ou en tube, notamment un gel douche, de shampoing, de lait, d'une émulsion, d'une microémulsion ou d'une nanoémulsion, notamment huile-dans-eau ou eau-dans-huile ou multiple ou siliconée, d'une lotion, notamment en flacon de verre, de plastique ou en flacon doseur ou en aérosol, d'une ampoule, d'un savon liquide, d'un pain dermatologique, d'une pommade, d'une mousse, d'un produit anhydre, de préférence liquide, pâteux ou solide, par exemple sous forme de bâtonnet, des poudres.

L'excipient pourra être choisi parmi le groupe consistant en les conservateurs, les émollients, les émulsifiants, les tensioactifs, les hydratants, les épaississants, les conditionneurs, les agents matifiants, les stabilisants, les antioxydants, les agents de texture, les agents de brillance, les agents filmogènes, les solubilisants, les pigments, les colorants, les parfums et les filtres solaires. Ces excipients sont de préférence choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxidases, les stabilisants à base de sucrose, les vitamines E et ses dérivés, les cires naturelles et synthétiques, les huiles végétales, les triglycérides, les insaponifiables, les phytosterols, les esters végétaux, les silicones et ses dérivés, les hydrolysats de protéines, l'huile de Jojoba et ses dérivés, les esters lipo/hydrosolubles, les betaines, les aminoxides, les extraits de plantes les esters de Saccharose, les dioxydes de Titane, les glycines, et les parabens, et encore de préférence parmi le groupe consistant en le butylène glycol, le stéareth-2, le stéareth-21, le glycol-15 stéaryl éther, le cétéaryl alcool, le phénoxyéthanol, le méthylparaben, l'éthylparaben, le propylparaben, le butylparaben, le butylène glycol, les tocopherols naturels, la glycérine, le dihydroxycetyl sodium phosphate, l'isopropyl hydroxycétyl éther, le glycol stéarate, le triisononanoin, l'octyl cocoate, le polyacrylamide, l'isoparaffine, le laureth-7, un carbomer, le propylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, Beeswax, les glycérides d'huile de coeur de palme hydrogénée, les glycérides d'huile de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée.

Dans un autre mode de réalisation, l'extrait selon l'invention est contenu dans une composition nutraceutique administrable par voie orale. Ainsi, la composition nutraceutique pourra se présenter sous la forme de poudre, gélules, capsules, nanocapsules, fibres alimentaires, gel alimentaire ou solution.

La composition cosmétique ou nutraceutique pourra contenir en outre un ou plusieurs autres ingrédients actifs sur la flore microbienne cutanée et/ou mucosale et/ou actifs sur la fonction barrière de la peau, parmi lesquels un oligosaccharide obtenu par synthèse enzymatique commercialisé par la société Solabia sous le nom de BioEcolia^{™} ou un complexe d'alpha-glucooligosaccharides commercialisé par la même société sous le nom de Ecoskin^{™}, un extrait d'*Alisma plantago-aquatica,* un extrait d'*Argania spinosa* (Lipofructyl^{™} Argan), un mélange de céramides (Sphingoceryl^{™} VEG), des cires de mangues sauvages (Irwinol^{™}) , des extraits purifiants de Boldo (Betapur^{™}), de Moringa (Freshaxyl^{™}), des produits à base d'inuline ou de fructooligosaccharides, des extraits de bifidobactéries ou encore un extrait *d'Orthosiphon stamineus* pour lutter contre la peau grasse (MAT-XS^{™} Bright).

La composition pourra par ailleurs contenir un ou plusieurs agents anti-pollution tels qu'un extrait de feuilles d'*Argania spinosa* commercialisé sous le nom d'Arganyl^{™} ou un extrait de graines de *Moringa oleifera* commercialisé sous le nom de Purisoft^{™} par la demanderesse ou encore un extrait de racine d'*Eperua falcata* commercialisé sous le nom de Eperuline^{™}.

Des agents favorisant l'hydratation tels qu'un polysaccharide extrait de graines de *Cassia angustifolia* commercialisée sous le nom de Hyalurosmooth^{™} par la demanderesse, ou un agent choisi parmi une des combinaisons contenant du pullulan, du hyaluronate de sodium et de l'alginate de sodium commercialisé sous le nom de PatcH2O^{™} par la demanderesse ou encore un ou plusieurs des composés du facteur natural d'hydratation (Natural Moisturizing Factor) ou un extrait naturel de miel commercialisé par la demanderesse sous le nom de Melhydran^{™} pourront être ajoutés à la composition cosmétique.

La composition cosmétique ou nutraceutique pourra aussi contenir un ou plusieurs actifs anti-âge conduisant à un effet complémentaire de l'extrait selon l'invention. On citera ainsi des actifs stimulant la synthèse des macromolécules du derme ou empêchant leur dégradation, des agents stimulant la prolifération des kératinocytes, des agents apaisants ou encore des agents actifs sur la régulation de la taille des pores et/ou leur ouverture, parmi lesquels :
- un agent stimulant la synthèse de fibronectine, en particulier un extrait de maïs, un tel extrait étant notamment commercialisé par BASF Beauty Care Solutions France sous le nom Deliner^{™} et le palmitoyl pentapeptide commercialisé par la société SEDERMA sous la dénomination commerciale Matrixil^{®},
- un agent stimulant la formation des fibres élastiques tel qu'un extrait d'*Origanum majorana* commercialisé sous le nom Dermagenist^{™} par la Demanderesse,
- un agent stimulant l'expression du perlécane et du dystoglycane dans la matrice extracellulaire et/ou dans la membrane basale épithéliale comme par exemple un extrait de *Polygonum bistorta* commercialisé sous le nom Perlaura^{™} par BASF Beauty Care Solutions France,
- un agent de protection du facteur de croissance des fibroblastes (FGF2) de la matrice extracellulaire contre sa dégradation et/ou sa dénaturation, notamment un extrait d'*Hibiscus Abelmoscus* tel que décrit dans la demande de brevet au nom de la BASF Beauty Care Solutions France déposée sous le numéro FR0654316 et commercialisé par BASF Beauty Care Solutions France sous le nom Linefactor^{™} et/ou un agent de stimulation de croissance des fibroblastes par exemple un extrait de soja fermenté contenant des peptides, connu sous le nom de Phytokine^{™} commercialisé par BASF Beauty Care Solutions France et également décrit dans la demande de brevet EP1119344B1 (Laboratoires Expanscience), et préférentiellement une combinaison de ces deux extraits,
- un agent stimulant la synthèse de laminine, en particulier un extrait de malt modifié par biotechnologie, un tel extrait étant notamment commercialisé par BASF Beauty Care Solutions France sous le nom Basaline^{™},
- un agent stimulant l'expression et/ou l'activité de la Hyaluronane synthase 2 (HAS2) tels que les extraits végétaux décrits dans la demande de brevet FR2 893 252 A1 et en particulier un extrait aqueux de Galanga (*Alpinia galanga*) et commercialisé par BASF Beauty Care Solutions France sous le nom Hyalufix^{™},
- un agent stimulant la synthèse de lysyl oxydase like (LOXL) tel qu'un extrait de *Geophila cordifolia* et ceux décrits dans la demande de brevet FR2855968, et en particulier un extrait d'aneth et commercialisé par BASF Beauty Care Solutions France sous le nom Lys'lastine ^{™},
- un agent stimulant la synthèse d'ATP intracellulaire, notamment un extrait d'algue *Laminaria digitata,*
- un actif inhibiteur des métalloprotéinases (MMP) telles que plus particulièrement les MMP 1, 2, 3, 9 tel que les rétinoïdes et dérivés, les oligopeptides et les lipopeptides, les lipoaminoacides, le lycopène, les isoflavones, la quercetine, le kaempferol, l'apigénine.
- un agent de regonflement notamment les sphères de comblement d'acide hyaluronique commercialisé par BASF Beauty Care Solutions France sous le nom de Hyaluronic Filling Spheres ^{™},
- un agent pour augmenter l'expression de LOX pour augmenter l'architecture de l'épiderme comme par exemple un extrait de *Cichorium intybus* commercialisé sous le nom de LOX-AGE^{™} par BASF Beauty Care Solutions France,
- un agent pour augmenter la déglycation du collagène et/ou augmenter l'expression du collagène de type I tel qu'une combinaison d'un extrait de feuilles de *Salvia miltiorrhiza* et de niacine commercialisé par BASF Beauty Care Solutions France sous le nom CollRepair^{™},
- un agent stimulant la synthèse de lumican et de collagène tel qu'un tétrapeptide synthétique acetyl Gln Asp Val His commercialisé par BASF Beauty Care Solutions France sous le nom Dermican^{™} et décrit dans la demande de brevet WO2005120554A1,
- un agent de protection et de stimulation de l'élastine, du collagène comme l'extrait de feuilles de *Manilkara multinervis* commercialisé par BASF Beauty Care Solutions France sous le nom Elestan^{™} et l'extrait de racine d'*Eperua falcata* commercialisé par BASF Beauty Care Solutions France sous le nom Eperuline^{™}
- un agent anti-taches pigmentaire notamment par inhibition de synthèse de mélanine tel que le complexe synergique d'extrait de *Pisum sativum* et de sucrose dilaurate commercialisé par BASF Beauty Care Solutions France sous le nom Actiwhite^{™}, ou l'acide hydroxyphenoxy propionique commercialisé par BASF Beauty Care Solutions France sous le nom Radianskin^{™}.

Un dernier objet de l'invention concerne encore un extrait de *S. cerevisiae,* qui est un hydrolysat fermenté de *S. cerevisiae,* fermenté par *L. plantarum,* ou une composition pharmaceutique, préférentiellement dermatologique, le comprenant, pour son utilisation pharmaceutique, préférentiellement dermatologique, encore préférentiellement par voie topique, dans le traitement ou la prévention des peaux et/ou muqueuses irritées, de pathologies impliquant une diminution de la teneur en microorganismes commensaux cutanés et/ou mucosaux et/ou une augmentation de la teneur en microorganismes pathogènes, telles que dermatites atopiques infectées, eczéma sévère, ulcères, plaies, herpès, acné surinfecté, dermatophytoses, candidoses. On entend au sens de la présente invention par « peaux et/ou muqueuses irritées » des peaux ou des muqueuses réactives impliquant un processus inflammatoire, notamment la libération de cytokines pro-inflammatoires.

L'extrait selon l'invention est donc utilisé pour induire un effet apaisant au niveau de la peau et/ou des muqueuses.

De façon avantageuse, on entend au sens de l'invention par « le traitement ou la prévention des peaux et/ou muqueuses irritées » une diminution du processus inflammatoire au niveau de la peau et/ou des muqueuses, notamment induite par les bactéries pathogènes, de façon particulière par *S. aureus.*

Dans un mode de réalisation préférentiel de l'invention, il s'agit d'une diminution de la libération de cytokines pro-inflammatoires, préférentiellement les interleukines IL6, d'au moins 20%, préférentiellement d'au moins 40%, encore préférentiellement d'au moins 65%, au niveau des kératinocytes, avantageusement de kératinocytes cultivés en présence de *S. aureus,* tel que décrit dans l'exemple 5.

Dans un mode de réalisation de l'invention, l'extrait est contenu dans la composition pharmaceutique, préférentiellement dermatologique, à une concentration en poids de 1×10⁻⁴ % à 10% par rapport au poids total de la composition, avantageusement de 1×10⁻³ % à 3%, encore avantageusement de 0,01 % à 3% en poids par rapport au poids total de la composition. Avantageusement, la composition comprend au moins un excipient dermatologiquement ou pharmaceutiquement acceptable.

Des exemples qui font référence à la description de l'invention sont présentés ci-après. Ces exemples sont donnés à titre d'illustration et ne sauraient limiter en aucun cas la portée de l'invention. Chacun des exemples a une portée générale. Les exemples font partie intégrante de la présente invention et toute caractéristique apparaissant nouvelle par rapport à un état de la technique antérieure quelconque à partir de la description prise dans son ensemble, y compris les exemples, fait partie intégrante de l'invention.

### Exemple 1 : Obtention d'un extrait de Saccharomyces cerevisiae.

### Exemple 1a)

Une quantité en poids de 3,5% de poudre de levure *S. cerevisiae* inactivée (non vivante) par rapport au poids total de la poudre et de l'eau comme solvant a été solubilisée et hydrolysée en présence de papaine (2,5 % v/v), à une température de 60°C durant une période de 72 heures sous agitation puis centrifugé (Vukasinovic Milic *et al.,* 2007). Le surnageant obtenu a ensuite été incubé en présence de la souche *Lactobacillus plantarum* jusqu'à épuisement du milieu nutritif contenant du glucose. L'extrait brut ainsi fermenté obtenu a été filtré (0,22µm) et la souche vivante de *L. plantarum* a été éliminée du milieu par filtration et l'extrait a été dilué dans du butylène glycol (20% p/p) et du pentylène glycol (5% p/p). Cet ingrédient a servi à démontrer sa capacité à augmenter le ratio *Staphylococcus epidermidis* / *Staphylococcus aureus* (Exemple 2).

### Exemple 1b)

Une quantité en poids de 5% de poudre de levure *S. cerevisiae* inactivée (non vivante) par rapport au poids total de la poudre et de l'eau comme solvant a été solubilisée et hydrolysée avec une hydrolyse enzymatique avec de la lyticase (0,02 % (v/v) à une température de 55°C durant une période de 72 heures sous agitation puis centrifugé (Vukasinovic Milic *et al.,* 2007). Le surnageant obtenu a été incubé en présence de la souche *L. plantarum* jusqu'à épuisement du milieu nutritif contenant du glucose. L'extrait brut ainsi fermenté a été filtré (0,22µm) et la souche vivante de *L. plantarum* a été éliminée du milieu.

### Exemple 1c)

Une quantité en poids de 3,5% de poudre de levure *S. cerevisiae* inactivée (non vivante) par rapport au poids total de la poudre et de l'eau comme unique solvant a été solubilisée et hydrolysée par autolyse de la souche dans l'eau, sous agitation et à une température de 52°C durant une période de 72 heures (Vukasinovic Milic *et al.,* 2007). Après centrifugation, le surnageant obtenu a été incubé en présence de la souche *L. plantarum* jusqu'à épuisement du milieu nutritif contenant du glucose. L'extrait brut ainsi fermenté a été filtré (0,22µm) et la souche vivante de *L. plantarum* a été éliminée du milieu.

### Exemple 2 : Exemple d'ingrédient cosmétique comprenant un extrait de Saccharomyces cerevisiae.

L'extrait de *Saccharomyces cerevisiae* correspond à l'extrait obtenu selon l'exemple 1a)*. Ingrédient cosmétique

| | |
|---|---|
| Butylène glycol | 10-25% |
| Pentylène glycol | 5-10% |
| Extrait * | >50% |

### Exemple 3 : Effet d'un extrait de Saccharomyces cerevisiae sur la flore microbienne cutanée.

### Exemple 3a) : Augmentation du ratio Staphylococcus epidermidis / Staphylococcus aureus

*Protocole* : Une crème contenant 2% en poids de l'ingrédient cosmétique tel que décrit dans l'exemple 2 par rapport au poids total de la crème a été appliquée 2 fois par jour durant 14 jours sur le visage d'une population de 10 femmes âgées de 18 à 45 ans de phototype caucasien. La mesure du ratio de la teneur de *Staphylococcus epidermidis* par rapport à la teneur de *Staphylococcus aureus* a consisté à mesurer la concentration en ADN microbien à l'aide d'un kit d'extraction (ADN MasterPure extraction kit), après prélèvement d'un échantillon de microorganismes de la peau du visage traité, par la technique dite de Swabbing (coton tige).

Chaque échantillon prélevé de la peau a été dilué dans un tampon de lyse contenant de la protéinase. Le milieu a été incubé à une température de 65°C durant une période de 15 minutes puis refroidi. De la RNAse a été ajoutée au milieu et le milieu a été incubé à une température de 37°C durant 30 minutes. L'ADN a ensuite été précipité puis le milieu centrifugé (4°C, 10 minutes). Le surnageant a été éliminé et le résidu lavé avec de l'éthanol. L'ADN extrait a été mesuré par PCR quantitative (kit TaqMan) à l'aide d'amorces spécifiques des gènes *sodA* et *femB* respectivement pour les souches *S*. *epidermidis* et *S. aureus* dans les conditions ci-après : chaque ADN a été analysé en double. Le programme de PCR a inclus une première étape d'activation de l'ADN polymérase à 95°C, suivi de 40 cycles de dénaturation / hybridation / extension. Les ARN messagers spécifiques des 2 gènes d'intérêt et du gène de référence ont été amplifiés et quantifiés en présence des amorces qui figurent dans le Tableau 1.

Les résultats bruts ont été rapportés au nombre total d'ADN bactérien en utilisant l'expression du gène de référence *ARN 16S* afin de normaliser les résultats.

**Tableau 1**

| Gènes | Séquences ADN amorces | Longueur amplicon (pb) |
|---|---|---|
| *sodA* sens (SEQ-1) | 5'-TCAGCAGTTGAAGGGACAGAT-3' | 135 |
| *sodA* antisens (SEQ-2) | 5'-CCAGAACAATGAATGGTTAAGG-3' | |
| *femB* 5' sens (SEQ-3) | 5'-TTACAGAGTTAACTGTTACC-3' | 651 |
| *femb* 3' antisens (SEQ-4) | 5'-ATACAAATCCAGCACGCTCT-3' | |
| *ARN 16S* sens (SEQ-5) | 5'- AGAGTTTGATCCTGGCTCAG-3' | 352 |
| *ARN16S* antisens (SEQ-6) | 5'-TGCTGCCTCCCGTAGGAGT-3' | |

*Résultats* : Les résultats sont présentés dans le Tableau 2 et exprimés par la moyenne du ratio de *S.epidermidis* /*S.aureus* (n = 10) en fonction inverse de log 10, aux temps T0 (D0) et T14jours (D14).

**Tableau 2**

| | MOY (Moyenne) | ET (Ecart Type) |
|---|---|---|
| D0 | 37,15 | 3,63 |
| D14 | 87,09 | 9,33 |

*Conclusion :* les résultats ont montré que l'extrait de *S. cerevisiae* selon l'invention a augmenté le ratio de la teneur de *Staphylococcus epidermidis* par rapport à la teneur de *S. aureus* d'un facteur 2,3 en 14 jours dans les peaux analysées, montrant donc que l'extrait selon l'invention a la capacité d'augmenter la flore microbienne commensale cutanée, en particulier la teneur en *S. epidermidis.*

### Exemple 3b) : Mise en évidence de l'absence d'activité antimicrobienne de l'extrait de Saccharomyces cerevisiae sur Staphylococcus aureus.

*Protocole* : La souche *S. aureus* a été cultivée *in vitro* en plaque multipuits dans un milieu de culture approprié. Après dilution au 1/10^{ème} d'un inoculum calibré de la souche *S. aureus* (2.105 CFU/mL) dans le milieu de culture lui-même dilué au ¼, l'ingrédient cosmétique tel que décrit dans l'exemple 2 a été ajouté au milieu aux concentrations finales de 0,5%, 1% et 2% en poids par rapport au poids total de l'ingrédient et du milieu de culture. Les plaques ont été incubées à 35°C en milieu aérobie durant une période de 24h et la densité optique corrélée à la croissance bactérienne a été lue à 600nm. Une diminution de la densité optique traduisant une diminution de la croissance bactérienne.

La densité optique a été mesurée dans les mêmes conditions dans le milieu de culture contenant la souche *S. aureus* sans ajout de l'extrait selon l'invention (Contrôle).

*Résultats* : Les résultats sont présentés dans le tableau 3 (n=3)

**Tableau 3**

| | MOY | ET |
|---|---|---|
| DO₆₀₀ₙₘ - Contrôle | 0.625 | 0.045 |
| DO₆₀₀ₙₘ - Extrait 0,5% (p/p) | 0,669 | 0,029 |
| DO₆₀₀ₙₘ - Extrait 1 % (p/p) | 0,737 | 0,039 |
| DO₆₀₀ₙₘ - Extrait 2% (p/p) | 0,694 | 0,032 |

*Conclusion* : les résultats ont montré une variation négligeable de la croissance bactérienne de la souche S. *aureus,* montrant que l'extrait de *S. cerevisiae* selon l'invention n'induit pas une diminution de la teneur de cette souche au niveau de la peau, donc n'a pas d'activité antimicrobienne, confirmant que l'augmentation du ratio de la teneur en *S. epidermidis*/*S. aureus* est due à une augmentation de la teneur en *S. epidermidis* et pas à une diminution de la teneur en *S. aureus.*

### Exemple 4 : Compositions cosmétiques comprenant un extrait de Saccharomyces cerevisiae selon l'invention

On procède selon les méthodes connues de l'homme de l'art pour mélanger ensemble les différentes parties A, B, C, D, E, ou F pour préparer une composition selon la présente invention. Les « produits de l'invention* » correspondent à l'ingrédient cosmétique selon l'exemple 2.
Formulation 4a : Composition cosmétique

| | | |
|---|---|---|
| A | Eau | qsp 100 |
| | Butylène Glycol | 2 |
| | Glycérine | 3 |
| | Sodium Dihydroxycetyl | 2 |
| | Phosphate, Isopropyl Hydroxycetyl Ether | |
| B | Glycol Stéarate SE | 14 |
| | Triisononaoin | 5 |
| | Octyl Cocoate | 6 |
| C | ButyleneGlycol, Methylparaben, Ethylparaben, Propylparaben, pH ajusté à 5,5 | 2 |
| D | Produits de l'invention* | 0,01 - 10 % |

Formulation 4b) :

| | | |
|---|---|---|
| A | Eau | qsp 100 |
| | Butylene Glycol | 2 |
| | Glycérine | 3 |
| | Polyacrylamide, Isoparafin, Laureth-7 | 2,8 |
| B | ButyleneGlycol, Methylparaben, | 2 |
| | Ethylparaben, Propylparaben ; | 2 |
| | Phenoxyethanol, Methylparaben, Propylparaben, Butylparaben, Ethylparaben | 0,5 |
| D | Butylene Glycol Produits de l'invention* | 0,01 - 10 % |

Formulation 4c)

| | | |
|---|---|---|
| A | Carbomer | 0,50 |
| | Propylene Glycol | 3 |
| | Glycerol | 5 |
| | Eau | qsp 100 |
| B | Octyl Cocoate | 5 |
| | Bisabolol | 0,30 |
| | Dimethicone | 0,30 |
| C | Sodium Hydroxide | 1,60 |
| D | Phenoxyethanol, Methylparaben, Propylparaben, Butylparaben, Ethylparaben | 0,50 |
| E | Parfum | 0,30 |
| F | Produits de l'invention* | 0,01 - 10 % |

### Exemple 5 : Effet apaisant de l'extrait selon l'invention

*Protocole* : Des kératinocytes humains ont été incubés durant une période de 3 jours à 37°C sous 5% de CO₂ et 95% d'humidité relative dans un milieu de culture DMEM (Dulbecco's Modified Eagle Medium) additionné de sérum de veau foetal (10%) contenant par ailleurs des antibiotiques. Le milieu de culture a ensuite été remplacé par 1mL de milieu EMEM (Eagle Minimum Essential Medium). Les cellules ont été incubées en présence de l'extrait *S*. *cerevisiae* préparé selon l'exemple 1a) à deux concentrations finales en poids par rapport au poids total du milieu et de l'extrait, durant une période de 24 heures. Le même milieu a été incubé sans extrait et a servi de contrôle.

Une quantité de 0,1mL de suspension bactérienne de *S. aureus* (ATCC12600) (1,5×10⁷) a été cultivée durant une période de 2h à 37°C (5% CO₂) en aérobie sous atmosphère saturée puis ajoutée au milieu de culture des kératinocytes en présence en présence de l'extrait selon l'invention. L'incubation a été poursuivie durant une période de 24 heures supplémentaires. La libération des cytokines inflammatoires IL6 et IL8 a ensuite été dosée par technique ELISA. Les résultats sont respectivement présentés dans les tableaux 4 et 5 (n = 3).

**Tableau 4 :**

| | Moyenne IL6 (%) | Ecart type |
|---|---|---|
| Contrôle + *S. aureus* | 100 | 8 |
| Extrait de *S. Cerevisiae* (0,5% p/p) + *S. aureus* | 53 | 4 |
| Extrait de *S. Cerevisiae* (1% p/p) + *S. aureus* | 32 | 5 |

**Tableau 5 :**

| | Moyenne IL8 (%) | Ecart type |
|---|---|---|
| Contrôle + *S. aureus* | 100 | 4 |
| Extrait de *S. Cerevisiae* (0,25% p/p) + *S. aureus* | 64 | 6 |
| Extrait de *S. Cerevisiae* (0,5% p/p) + *S. aureus* | 46 | 3 |
| Extrait de *S. Cerevisiae* (1% p/p) + *S. aureus* | 39 | 2 |

*Conclusion* : L'extrait selon l'invention a induit une diminution de la libération de cytokines IL6 et IL8 induites par *S. aureus* dans des kératinocytes en culture. Ceci démontre l'effet apaisant de l'extrait au niveau de la peau et/ou des muqueuses, donc son intérêt dans la prévention et/ou le traitement des peaux et/ou des muqueuses irritées.

## Revendications

1. Utilisation cosmétique et/ou nutraceutique non-thérapeutique d'un extrait de *Saccharomyces cerevisiae* pour augmenter la flore microbienne commensale cutanée et/ou mucosale humaine, dans laquelle l'extrait de *Saccharomyces cerevisiae* est un hydrolysat fermenté par Lactobacillus plantarum.

2. Utilisation selon la revendication 1, dans laquelle l'extrait de *Saccharomyces cerevisiae* augmente la teneur en *Staphylococcus epidermidis* au niveau de la peau et/ou des muqueuses.

3. Utilisation selon l'une quelconque des revendications 1 et 2, dans laquelle l'extrait est administré par voie topique et/ou orale.

4. Utilisation de l'extrait de *Saccharomyces cerevisiae* selon l'une quelconque des revendications 1 à 3, dans laquelle l'extrait est contenu dans une composition cosmétique et/ou nutraceutique comprenant au moins un excipient cosmétiquement ou nutraceutiquement acceptable, à une concentration de 1x10⁻⁴% à 10%, avantageusement de 1x10⁻³% à 3%, avantageusement de 0,01% à 3% en poids par rapport au poids total de la composition.

5. Utilisation selon l'une quelconque des revendications 1 à 4 pour augmenter le ratio de la teneur d'une ou plusieurs souches microbiennes commensales par rapport à la teneur d'une ou plusieurs souches microbiennes pathogènes au niveau de la peau et/ou des muqueuses, de préférence pour augmenter le ratio de la teneur de *Staphylococcus epidermidis* par rapport à la teneur de *Staphylococcus aureus* au niveau de la peau et/ou des muqueuses.

6. Utilisation selon l'une quelconque des revendications 1 à 5, dans laquelle l'extrait améliore l'homéostasie cutanée, avantageusement au niveau de la peau et/ou des muqueuses.

7. Utilisation selon l'une quelconque des revendications 1 à 5, **caractérisée en ce que** l'extrait diminue les manifestations inesthétiques et/ou inconfortables des peaux et/ou muqueuses sensibles.

8. Utilisation selon l'une quelconque des revendications 1 à 7, **caractérisée en ce que** l'extrait est appliqué sur tout ou partie du corps humain choisi parmi les jambes, les pieds, les aisselles, les mains, les cuisses, le ventre, le décolleté, le cou, les bras, le torse, le dos, la muqueuse labiale, le visage et/ou le cuir chevelu, avantageusement le décolleté et/ou le visage.

9. Extrait de *Saccharomyces cerevisiae* pour son utilisation pharmaceutique, préférentiellement dermatologique, dans le traitement ou la prévention des peaux et/ou muqueuses irritées et/ou de pathologies impliquant une diminution de la teneur en microorganismes commensaux cutanés et/ou mucosaux et/ou une augmentation de la teneur en microorganismes pathogènes, telles que dermatites atopiques infectées, eczéma sévère, ulcères, plaies, herpès, acné surinfecté, dermatophytoses, candidoses, l'extrait étant un hydrolysat de *Saccharomyces cerevisiae* fermenté par Lactobacillus plantarum.

10. Extrait pour une utilisation selon la revendication 9, tel que l'extrait est présent dans une composition dermatologique ou pharmaceutique à une concentration en poids de 0,1% à 3%, avantageusement de 1% à 3% en poids par rapport au poids total de la composition.

## Patentansprüche

1. Kosmetische und/oder nutrazeutische nichttherapeutische Verwendung eines Extrakts von Saccharomyces cerevisiae zur Erhöhung der menschlichen mikrobiellen kommensalen Haut- und/oder Schleimhautflora, wobei der Extrakt von Saccharomyces cerevisiae ein durch Lactobacillus plantarum fermentiertes Hydrolysat ist.

2. Verwendung nach Anspruch 1, wobei der Extrakt von Saccharomyces cerevisiae den Gehalt an Staphylococcus epidermidis an der Haut und/oder den Schleimhäuten erhöht.

3. Verwendung nach einem der Ansprüche 1 und 2, wobei der Extrakt topisch und/oder oral verabreicht wird.

4. Verwendung des Extrakts von Saccharomyces cerevisiae nach einem der Ansprüche 1 bis 3, wobei der Extrakt in einer kosmetischen und/oder nutrazeutischen Zusammensetzung, die mindestens einen kosmetisch oder nutrazeutisch akzeptablen Exzipienten umfasst, in einer Konzentration von 1 x 10⁻⁴ Gew.-% bis 10 Gew.-%, vorteilhafterweise von 1 x 10⁻³ Gew.-% bis 3 Gew.-%, vorteilhafterweise von 0,01 Gew.-% bis 3 Gew.-%, bezogen auf das Gesamtgewicht der Zusammensetzung, enthalten ist.

5. Verwendung nach einem der Ansprüche 1 bis 4 zur Erhöhung des Verhältnisses des Gehalts eines oder mehrerer kommensaler Mikrobenstämme in Bezug auf den Gehalt eines oder mehrerer pathogener Mikrobenstämme an der Haut und/oder den Schleimhäuten, vorzugsweise zur Erhöhung des Verhältnisses des Gehalts an Staphylococcus epidermidis in Bezug auf den Gehalt an Staphylococcus aureus an der Haut und/oder den Schleimhäuten.

6. Verwendung nach einem der Ansprüche 1 bis 5, wobei der Extrakt die Hauthomöostase, vorteilhafterweise an der Haut und/oder den Schleimhäuten, verbessert.

7. Verwendung nach einem der Ansprüche 1 bis 5, **dadurch gekennzeichnet, dass** der Extrakt unästhetische und/oder unangenehme Manifestationen von empfindlicher Haut und/oder Schleimhaut verringert.

8. Verwendung nach einem der Ansprüche 1 bis 7, **dadurch gekennzeichnet, dass** man den Extrakt auf den gesamten oder einen Teil des menschlichen Körpers, ausgewählt aus Beinen, Füßen, Achselhöhlen, Händen, Oberschenkeln, Bauch, Dekolleté, Hals, Armen, Rumpf, Rücken, Lippenschleimhaut, Gesicht und/oder Kopfhaut, vorteilhafterweise aus Dekolleté und/oder Gesicht, anwendet.

9. Extrakt von Saccharomyces cerevisiae zur pharmazeutischen, vorzugsweise dermatologischen, Verwendung, bei der Behandlung oder Vorbeugung von gereizter Haut und/oder Schleimhaut und/oder von Pathologien, die eine Verringerung des Gehalts an kommensalen Haut- und/oder Schleimhautmikroorganismen und/oder eine Erhöhung des Gehalts an pathogenen Mikroorganismen beinhalten, wie infizierte atopische Dermatitiden, schweres Ekzem, Geschwüre, Wunden, Herpes, superinfizierte Akne, Dermatophytosen, Candidosen, wobei der Extrakt ein durch Lactobacillus plantarum fermentiertes Hydrolysat von Saccharomyces cerevisiae ist.

10. Extrakt zur Verwendung nach Anspruch 9, wobei der Extrakt in einer dermatologischen oder pharmazeutischen Zusammensetzung in einer Konzentration von 0,1 Gew.-% bis 3 Gew.-%, vorteilhafterweise von 1 Gew.-% bis 3 Gew.-%, bezogen auf das Gesamtgewicht der Zusammensetzung, vorliegt.

## Claims

1. Non-therapeutic cosmetic and/or nutraceutical use of a *Saccharomyces cerevisiae* extract for increasing the human cutaneous and/or mucosal commensal microbial flora, wherein the *Saccharomyces cerevisiae* extract is a hydrolysate fermented by *Lactobacillus plantarum.*

2. Use according to Claim 1, wherein the Saccharomyces cerevisiae extract increases the amount of Staphylococcus epidermidis at the skin and/or the mucous membranes.

3. Use according to either one of Claims 1 and 2, wherein the extract is administered topically and/or orally.

4. Use of the Saccharomyces cerevisiae extract according to any one of Claims 1 to 3, wherein the extract is contained in a cosmetic and/or nutraceutical composition comprising at least one cosmetically or nutraceutically acceptable excipient at a concentration of 1×10⁻⁴% to 10%, advantageously of 1×10⁻³% to 3%, advantageously of 0.01% to 3% by weight relative to the total weight of the composition.

5. Use according to any one of Claims 1 to 4 for increasing the ratio of the amount of one or more commensal microbial strains to the amount of one or more pathogenic microbial strains at the skin and/or the mucous membranes, preferably for increasing the ratio of the amount of Staphylococcus epidermidis to the amount of Staphylococcus aureus at the skin and/or the mucous membranes.

6. Use according to any one of Claims 1 to 5, wherein the extract improves the cutaneous homeostasis, advantageously at the skin and/or the mucous membranes.

7. Use according to any one of Claims 1 to 5, **characterized in that** the extract decreases unaesthetic and/or uncomfortable manifestations of sensitive skin and/or mucous membranes.

8. Use according to any one of Claims 1 to 7, **characterized in that** the extract is applied to all or part of the human body, chosen from the legs, feet, underarms, hands, thighs, stomach, chest, neck, arms, torso, back, labial mucous membrane, face and/or scalp, advantageously the chest and/or face.

9. *Saccharomyces cerevisiae* extract for the pharmaceutical, preferentially dermatological use thereof in the treatment or prevention of irritated skin and/or mucous membranes and/or of pathological conditions involving a decrease in the amount of cutaneous and/or mucosal commensal microorganisms and/or an increase in the amount of pathogenic microorganisms, such as infected atopic dermatitis, severe eczema, ulcers, wounds, herpes, superinfected acne, dermatophytosis or candidosis, the extract being a hydrolysate of *Saccharomyces cerevisiae* fermented by *Lactobacillus plantarum.*

10. Extract for use according to Claim 9, such that the extract is present in a dermatological or pharmaceutical composition at a concentration by weight of 0.1% to 3%, advantageously of 1% to 3% by weight relative to the total weight of the composition.
